Europäisches Patentamt

**European Patent Office**

Office européen des brevets

(11) Veröffentlichungsnummer: **0 101 063**

**B1**

(12) **EUROPÄISCHE PATENTSCHRIFT**

(45) Veröffentlichungstag der Patentschrift: **04.04.90**

(21) Anmeldenummer: **83107892.8**

(22) Anmeldetag: **10.08.83**

(51) Int. Cl.⁵: **C 07 K 7/04,** A 61 K 37/02, A 61 K 35/26

(54) **Neues Polypeptid mit Wirkung auf das Immunsystem, Verfahren zu seiner Isolierung und Reinigung, seine Verwendung und dieses enthaltende Mittel.**

(30) Priorität: **13.08.82 DE 3230151**

(43) Veröffentlichungstag der Anmeldung:
**22.02.84 Patentblatt 84/08**

(45) Bekanntmachung des Hinweises auf die
Patenterteilung:
**04.04.90 Patentblatt 90/14**

(84) Benannte Vertragsstaaten:
**AT BE CH DE FR GB IT LI LU NL SE**

(56) Entgegenhaltungen:
**EP-A-0 033 384**
**FR-A-2 492 663**
**US-A-4 010 148**
**US-A-4 079 127**

**"IMMUNOSTIMULATION", 1980, Ausgeber L. Chedid et al., Springer-Verlag, Berlin, DE; T.L.K. LOW et al.: "Thymosin and other thymic hormones and their synthetic analogues", Seiten 132-134**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Seipke, Gerhard, Dr.**
**Brunnenweg 4**
**D-6200 Wiesbaden (DE)**
Erfinder: **Tripier, Dominique, Dr.**
**Im Kirschgarten 18**
**D-6239 Eppstein/Taunus (DE)**
Erfinder: **Johnscher, Gerd, Dr.**
**Dingesweg 9**
**D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein bisher nicht bekanntes aus Thymusdrüsen isolierbares Polypeptid mit Wirkung auf das Immunsystem, das gekennzeichnet ist durch ein Molekulargewicht von 3480, durch eine UV-Absorption mit einem Maximum bei 205 bis 210 nm, durch einen isoelektrischen Punkt von 3,95±0,15 und durch folgende Aminosäurezusammensetzung:

4 Asparaginsäure, 3 Threonin, 3 Serin, 6 Glutaminsäure, 1 Glycin, 4 Alanin, 3 Valin, 1 Isoleucin, 1 Leucin, 5 Lysin, 1 Arginin,

wobei der N-Terminus gegebenenfalls eine Acylgruppe von 1 bis 6 Atomen oder eine Acylglycylgruppe, deren Acylgruppe 1 bis 6 Kohlenstoffatome aufweist, trägt, sowie dessen pharmakologisch unbedenklichen Salze.

Mit zellfreien Proteinextrakten aus Thymusdrüsen von Kälbern (z.B. die als Standard-Präparat bekannte Thymosin-Fraktion 5) lassen sich bekanntlich Immunmangelerscheinungen unterdrücken, die z.B. die Ursache verminderter Transplantat-Abstoßung, erhöhter Tumorbildungswahrscheinlichkeit, zunehmende Infektionsanfälligkeit und beschleunigter Alterungsprozesse sein können. Von der erfolgreichen klinischen Anwendung dieser Fraktion 5, insbesondere am Lungenkrebs-Patienten, wurde berichtet (P. B. Chrétien et al. Cancer Treat. Rep. 62 (1978) 1787—1790).

Es ist aus ersichtlichen Gründen wünschenswert, in einer Therapie nicht ein Substanzgemisch einzusetzen, das möglicherweise auch Inhibitoren des gewünschten Effektes oder sogar toxische Verbindungen enthält, sondern charakterisierte Reinsubstanzen definierter und reproduzierbarer Wirksamkeit zu verwenden. Noch dazu ist ein solches Substanzgemisch in der Zusammensetzung schwer zu standardisieren, da diese u.a. vom Alter der Schlachttiere abhängt. Beispiel für solche reinen Substanzen, die aus Gemischen isoliert wurden, sind Thymosin $\alpha_1$, $\beta_1$, $\beta_4$, $\beta_8$, $\beta_9$, wobei die erste der genannten Substanzen am besten hinsichtlich ihrer biologischen Wirkung untersucht wurde (T. L. K. Low et al. J. Biol. Chem. 254 (1979) 981—986, T. L. K. Low et al. Proc. Natl. Acad. Sci. USA 78 (1981) 1162—1166).

Nun wurde aber gerade über Thymosin $\alpha_1$, sowie $\beta_8$ in jüngster Zeit berichtet (E. Hannappel et al. Proc. Natl. Acad. Sci. USA 79 (1982) 1708—1711) und die vorliegende Arbeit zur Isolierung des erfindungsgemäßen Peptids unterstützt diese Befunde, daß beide Peptide nur als Folge einer nicht schonenden und damit schwer reproduzierbaren Isolierungstechnik resultieren (Thymosin $\alpha_1$ konnte in Nebenfraktionen bei der Isolierung des erfindungsgemäßen Hormons nicht nachgewiesen werden).

Der Erfindung lag daher die Aufgabe zugrunde, unter schonenden Bedingungen reine Hormone zu isolieren, um damit die intakten, natürlichen Wirkprinzipien zur Verfügung zu haben. Da es in der Thymusdrüse eine Vielzahl solcher Wirkprinzipien gibt und diese Vielfalt mit Sicherheit eine Bedeutung hat, z.B. in einer unterschiedlichen Wirkungsspezifität, ist die Reindarstellung aller dieser Substanzen zur Erstellung einer optimalen Palette zur Therapie der oben erwähnten Krankheiten von größter Bedeutung.

Das erfindungsgemäß isolierte Peptid weist eine hohe, vorzugsweise über 90 %ige Reinheit auf, wie sich durch in der Peptidchemie übliche Methoden leicht zeigen läßt. Zu diesen Methoden gehören Polyacrylamid-Gel-Elektrophorese, Isoelektrische Fokussierung und Aminosäureanalyse. Die neue Verbindung kann in unterschiedlich erfindungsgemäßen Aufarbeitsvarianten immer in gleichguter Ausbeute und mit der gleichen hohen Reinheit gewonnen werden, wobei die Produkte sämtlicher Aufarbeitsverfahren miteinander identisch sind. Dies läßt den Schluß zu, daß es sich um einen intakten Naturstoff handelt und nicht um ein aufarbeitungsbedingtes Spaltprodukt.

Das erfindungsgemäße Peptid ist weiß, geruchlos, bei höherer Temperatur z.B. 80°C in neutraler Lösung stabil und in trockener Form ohne Aktivitätsverlust über mindestens 3 Jahre bei Temperaturen unterhalb +5°C lagerfähig. Seiner chemischen Struktur nach ist es ein Polypeptid, in dem nach kompletter hydrolytischer Spaltung keine anderen Bestandteile außer den Aminosäuren nachzuweisen sind.

Bestimmt man die Aminosäurezusammensetzung nach der Methode von Moore und Stein (Methods of Enzymology Band VI, 819—831, herausgegeben von Colovick und Kaplan, Academic Press, New York, London, 1963) findet man folgende Werte:

4 Asparaginsäure, 3 Threonin, 3 Serin, 6 Glutaminsäure, 1 Glycin, 4 Alanin, 3 Valin, 1 Isoleucin, 1 Leucin, 5 Lysin, 1 Arginin.

Kennzeichnend für das erfindungsgemäße Polypeptid ist das Fehlen von Methionin, Cystein, Histidin, Phenylalanin, Tyrosin und Tryptophan. Die Abwesenheit insbesondere von Methionin und den aromatischen Aminosäuren kann bei den Anreicherungsverfahren zur Reinheitsbeurteilung der Fraktionen dienen. Sie liefert außerdem die nachträglich Erklärung dafür, daß keine exakte Korrelation zwischen den Peakflächen in den chromatographischen Trennungen und den aus den Peak's isolierten Substanzmengen besteht, wenn man die Detektion bei überlicherweise verwendeten Wellenlänge von 254 nm durchführt. Zur Molekulargewichtsbestimmung bedient man sich der SDS-Polyacrylamidgel-Elektrophorese (J. O. Thomas in: Techniques in Protein and Enzyme Biochemistry B 106, 102 ff (1978) Elsevier/North-Holland, Biomedical Press), wobei man einen Wert von etwa 3000—4000 erhält, wenn man die Laufstrecke des Polypeptids mit der von bekannten Referenzsubtanzen wie z.B. Trypsin-Inhibitor aus Sojabohnen (MG 6000) und Insulin-B-Kette (MG 2500) vergleicht. Dieses Ergebnis korreliert mit dem Befund der Aminosäureanalyse.

In der basischen Disc-Elektrophorese in Polyacrylamidgel (L. Ornstein, Ann. N.Y. Acad. Sci. 121 (1964) 321—349 und B. J. Davies, Ann. N.Y. Acad. Sci. 121 (1964) 404—427) zeigt das erfindungsgemäße Produkt im wesentlichen eine einzige Bande mit einem Rf-Wert von 0,5 bis 0,6. Ein ähnliches Muster zeigt die isoelektrische Fokussierung in 0,3 mm starken, nach der Technik von Radola (B. J. Radola, Elektrophorese *1* (1980) 43—46)) hergestellten Gelen, die Ampholyte für den pH-Bereich 2—9 und 6 M Harnstoff enthalten. Der isoelektrische Punkt des erfindungsgemäßen Polypeptids von 3,95±0,15 wird durch Vergleich mit kommerziell erhältlichen Testgemischen festgelegt; die angegebene Fehlerbreite kommt dadurch zustande, daß Probe und Vergleich niemals exakt gleiche Ionenstärken aufweisen und auch der Harnstoff, der für das Erreichen einer hohen Auflösung erforderlich ist, den isoelektrischen Punkt von Polypeptiden beeinflussen kann. Die Anfärbung derartiger Polyacrylamidgele geschieht mit einem modifizierten Coomassie-Blau (Blakesley und Boezi, Analytical Biochemistry *82* (1977) 580—582).

Die Erfindung betrifft weiterhin ein Verfahren zur Gewinnung dieses gereinigten Polypeptids, das durch Isolierung des Polypeptids aus Thymus mit hilfe einer Kombination von Fällungsmethoden, Membranfiltrationen und Chromatographie gekennzeichnet ist.

Die Thymusdrüsen werden vorzugsweise mit einer wäßrigen Lösung eines anorganischen Salzes mit puffernder Wirkung im pH Bereich von 5,0 bis 9,0, vorzugsweise Phosphat extrahiert.

Das unlösliche Material wird abgetrennt und das Polypeptid aus dem so erhaltenen Extrakt abgetrennt und isoliert.

Der chromatographische Schritt des obengenannten Gewinnungsverfahrens besteht vorzugsweise aus einer Kationen- und/oder Anionen-Austauschchchromatographie, einer Chromatographie an unspezifischen Adsorbentien, insbesondere Hydroxylapatit, einer Verteilungschromatographie, insbesondere Verteilung zwischen zwei nicht mischbaren Medien, wobei eines der Medien stark wasserhaltig ist, oder eine Kombination aus zwei oder mehr dieser Methoden.

Es ist von Vorteil, wenn man für diese Chromatographie einen Extrakt einsetzt, den man einer Hitzedenaturierung unterzogen hat, wobei das erfindungsgemäße Peptid gelöst bleibt und seine volle Wirksamkeit behält, während sich andere Proteine durch Sedimentation oder Filtration abtrennen lassen. Man kann für die Chromatographie auch ein Material einsetzen, das bei der Behandlung des Extrakts durch Zugabe von organischen Lösungsmitteln die mit Wasser mischbar sind, vorzugsweise Aceton, als Niederschlag abgetrennt wird. Ebenso kann ein Extrakt eingesetzt werden, den man einer fraktionierten Fällung durch Zugabe wasserlöslicher anorganischer und/oder organischer Salze, vorzugsweise Ammoniumsulfat, unterzogen hat; wobei in bestimmten, vom verwendeten Salz abhängigen Konzentrationsbereichen das Peptid ausfällt und durch Sedimentationstechniken angereichert wird. Weiterhin ist für die Chromatographie einen Extrakt verwendbar, der durch Fraktionierung mittels Membranfiltration und/oder Gelfiltration nur noch Peptide enthält, die kleiner sind als 10 000 Dalton.

Selbstverständlich könnten für die Chromatographie auch Extrakte eingesetzt werden, die man durch Kombination von zwei oder mehreren der vorstehend beschriebenen Verfahren erhalten hat.

Das Ausgangsmaterial für die Darstellung des erfindungsgemäßen Polypeptids sind frische Thymusdrüsen oder solches Drüsenmaterial, das rasch nach der Schlachtung tiefgekühlt eingelagert wurde. Aus diesem Material wird durch Zerkleinererung in eingefrorenen Zustand und Zugabe einer Salzlösung unter weiterer Kühlung (0—+4°C) ein wäßriger Extrakt bereitet. Es ist vorteilhaft, diesen Extrakt durch schnelle Erhitzung auf eine optimale Temperatur zwischen 70 bis 90°C, vorzugsweise 80°C zu unterziehen, weil dadurch die Hauptmenge der Proteasen inaktiviert und als Fällung abgetrennt wird. Die Abtrennung erfolgt durch Zentrifugation und Filtration über Gaze.

Es wurde durch Vergleich mit anderen Protease-Inaktivationsmethoden, wie Zusatz von chemischen Inaktivatoren, z.B. Phenylmethylsulfonylfluorid (PMSF), bewiesen, daß weder die Ausbeute noch die Aktivität bei alleiniger Hitzebehandlung absinkt, daß also dieses Verfahren für diesen Zweck an sich ausreichend ist.

Um zu kleineren Volumina zu gelangen und damit die weitere Aufarbeitung zu erleichtern, sollte aus dem hitzebehandelten Extrakt die interessierende Proteinfraktion durch Fällung angereichert werden. Diese Fällung kann in der Weise durchgeführt werden, daß man den Extrakt in ein organisches Lösungsmittel gibt, das mit Wasser mischbar ist. Es kann z.B. Aceton in einer mehrfachen Menge des Extraktvolumens, vorzugsweise der 5-fachen Menge, eingesetzt werden, wobei die Fällung bei sehr tiefen Temperaturen zwischen −10 und −30°C, vorzugsweise −20°C vorgenommen wird. Von Bedeutung ist ferner, daß man das Gemisch möglichst rasch filtriert oder zentrifugiert, da bei längerem Stehenlassen andere Begleitstoffe mitgefällt werden. Auch bei diesem Schritt werden möglicherweise in Lösung verbliebene Enzyme durch die hohe Konzentration an organischem Lösungsmittel inhibiert. Die so erhaltene Fällung ist ohne Aktivitätsverlust im gefrorenen Zustand lagerfähig.

Eine andere Möglichkeit zur Anreicherung durch Fällung, die entweder alternativ oder successiv zur vorgenannten Methode erfolgen kann, ist die Zugabe von neutralen Salzen, wie z.B. Ammoniumsulfat. Die Fällung kann durch pH-Steuerung eine gewisse Selektivität erreichen. Das erfindungsgemäße Peptid, das einen isoelektrischen Punkt von etwa 3,95 hat, kann im pH-Bereich zwischen 3 und 5, vorzugsweise 4, durch Zugabe von Ammoniumsulfat bis 50% Sättigung so ausgefällt werden, daß eine Vielzahl von Begleitproteinen dabei in Lösung bleibt. Auch diese Fällung wird unter Kühlung, vorzugsweise zwischen 0 und +4°C, durchgeführt.

Um weitere Proteine mit ähnlichen isoelektrischen Punkt, aber höherem Molekulargewicht abzutrennen, haben sich zwei Methoden bewährt: Gelfiltration und Ultrafiltration, wobei letztere für den technischen Maßstab besonders geeignet ist. Die Gelfiltration erlaubt eine Einteilung in engere molekulare Gewichtsbereiche, hat aber eine begrenztere Kapazität als die Ultrafiltration, so daß sie im Verlauf der Aufarbeitung größerer Extraktmengen eher für spätere Schritte, z.B. Nachreinigung von Ultrafiltrations- oder Säulenchromatographiefraktionen geeignet erscheint. Zur Gelfiltration bedient man sich am besten sphärischer Partikel auf der Basis von vernetztem Dextran, Polyacrylamid oder modifiziertem Kieselgel, z.B. Bio-Gel®, Sephadex®, Ultrogel® oder Polyol mit einem Fraktionierungsbereich bis 10 000 Dalton. Am besten wird die Gelfiltration, wie überhaupt alle weiteren Reinigungsschritte wegen des isoelektrischen Punktes des erfindungsgemäßen Produktes in wäßrigen Puffern bei pH-Werten zwischen 4 und 9 durchgeführt, da sonst die Löslichkeit des Polypeptids nicht gewährleistet ist.

Mit zwei Ausnahmen (CM-Cellulose, Hydroxylapatit, s.u.) hat sich der pH-Bereich 7,5—8,5 (Ammoniumhydrogencarbonat oder Tris/HCl) als besonders günstig erwiesen.

Die Ultrafiltration erfolgt am besten in zwei Stufen, damit die Membranen nicht zu hoch belastet werden (Verstopfung der Poren). Als günstig hat sich z.B. die Benutzung von einer Kapillarmembrane der Ausschlußgrenze 50 000 Dalton in Kombination mit einer Flachmembran der Ausschlußgrenze 10 000 Dalton erwiesen. Mit der Kapillarmembrane erreicht man eine schnelle Abtrennung von höhermolekularen Material, welches den Durchfluß der selektiver arbeitenden Flachmembran vermindern würde.

Das so erhaltene Material besteht auf Grund der bisher angewandten Methode immer noch aus einer Mischung von Polypeptiden. Auf Grund seines isoelektrischen Punktes bindet das erfindungsgemäße Polypeptid nicht an einen Kationenaustauscher bei dem üblicherweise benutzten pH-Bereich von 4 bis 5, so daß es mit Hilfe dieser Austauscher auf Basis von Cellulose, Dextran, Polyacrylamid, Polystyrolharz oder Kieselgel schnell von einem großen Teil des Begleitprodukte, die einen isoelektrischen Punkt über pH 4 bis 5 besitzen und deshalb absorbiert werden, abgetrennt werden kann. Diese Abtrennung kann in einer Chromatographiesäule oder in einem Batch-Verfahren erfolgen.

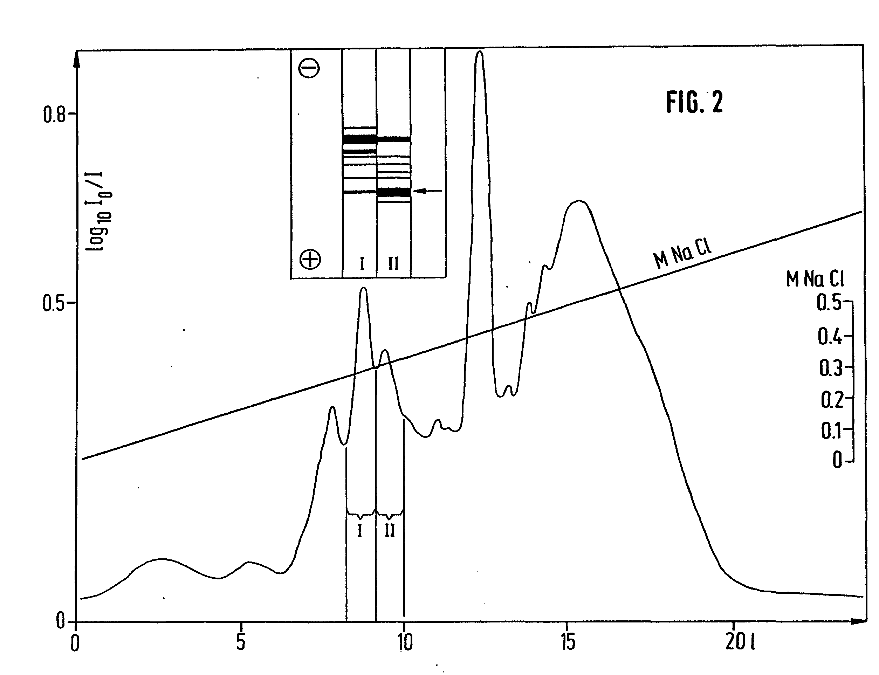

An einem Anionenaustauscher auf der Basis von Dextran, Polyacrylamid, Cellulose, Polystyrolharz oder Kieselgel und bei pH-Werten von 5 bis 9, vorzugsweise 8, wird das Peptid dagegen gebunden und kann durch Wahl geeigneter Bedingungen weitgehend selektiv eluiert werden Solche Bedingungen sind z.B. das Anlegen eines pH- oder Salzgradienten. Als günstig hat sich die Arbeit mit einem 50 millimolaren Tris-HCl-Puffer, pH 8 und einem Natriumchloridgradienten von 0 bis 0,5 M verteilt über das 5 bis 10-fache Säulenvolumen erwiesen. Vor dieser chromatographischen Trennung weist das Polypeptidgemisch in der basischen Gelektrophorese eine Vielzahl von kaum aufgelösten. Banden auf; bei Anfärbung mit Coomassieblau entsteht der Eindruck einer kontinuierlichen Untergrundfärbung mit nur sehr wenigen definierten Banden. Das erfindungsgemäße Polypeptid ist auf dieser Stufe nicht sichtbar. Erst die Analyse der Anionenaustauscherfraktion ergibt ein übersichtliches Bild. Fast jeder Peak der Chromatographie ist mit einer definierten Bande der Elektrophorese korrelierbar, so daß eine Lokalisierung des erfindungsgemäßen Peptids bei etwa 35% des in Beispiel 7 beschriebenen Gradienten möglich ist (s. Abbildung 2).

Wegen der Komplexität des Chromatogramms ist das Erreichen einer völligen Homogenität in diesem ersten Chromatographie-Schritt nicht gewährleistet. Durch Rechromatographie über das gleiche Trennsystem ist ein guter weiterer Reinigungseffekt zu erzielen. Auch ein Gelfiltration trennt noch eine kleine Menge höhermolekularen Materials ab. Der Reinheitsgrad wird hierbei aber nur geringfügig erhöht, da das Material dieses Trennprinzip schon intensiv durchlaufen hat (Ultrafiltration).

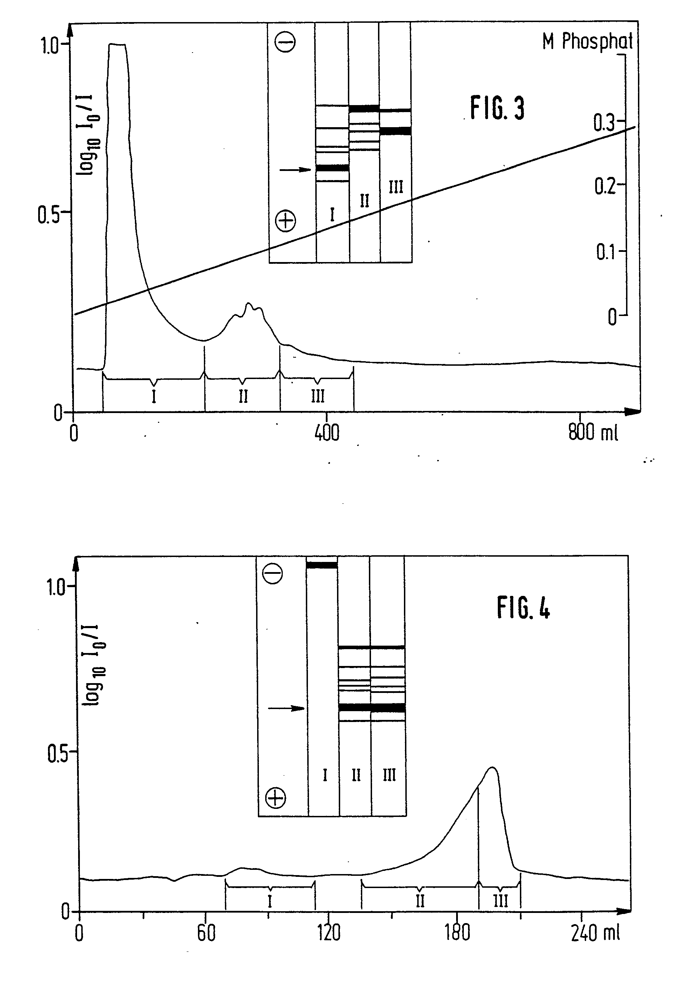

Vorteilhaft ist die Anwendung einer völlig anderen Methode, der Absorption an Hydroxylapatit, vor oder nach der Rechromatographie auf Ionenaustauscher, zumal dieses Verfahren verlustarm und rasch arbeitet, da das erfindungsgemäße Polypeptid in üblicher Weise benutzten 10 millimolaren Phosphatpuffer bei pH 6,8 die Trennsäule unretadiert durchläuft und nur Begleitstoffe gebunden werden (siehe Abbildung 3).

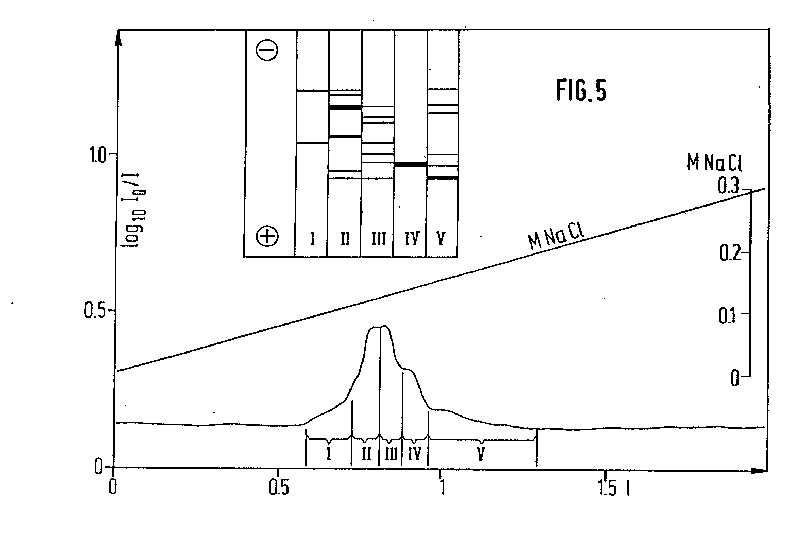

Im allgemeinen wurde die Chromatographie an Hydroxylapatit vor der endgültigen Reinigung durch Rechromatographie am Anionenaustauscher eingeschaltet, aber auch an anderer Stelle im aufarbeitungsschema (z.B. im Anschluß an den Kationenaustauscher) ist eine Verwendung dieser Methode mit gleichem Erfolg angewandt worden. Der Rechromatographieschritt an Anionenaustauscher erfolgt in einer Säule geringen Volumens und mit einem wesentlich flacheren Gradientenverlauf (s. Abbildung 5).

Es ist selbstverständlich, daß jedem Schritt der Reinigung eine Entsalzung und Lyophilisierung folgt. Lediglich das aus der Kationenaustauscher-Behandlung anfallende Material kann, da die enthaltene Salzmenge gering ist, direkt für den nächsten Schritt eingesetzt werden. Die Entfernung der Salze erfolgt entweder durch Ultrafiltration über eine geeignete Flachmembran mit der Ausschlußgrenze 500 Dalton oder 10 000 Dalton oder durch Gelfiltration über ein geeignetes Material auf der Basis von vernetztem Dextran oder Polyacrylamid, z.B. Sephadex G 10 oder G 25 oder Bio Gel P-2 oder Ultrogel ACa 202.

Am besten arbeitet man natürlich mit Puffern, die bei der nachfolgenden Lyophilisierung entfernt werden können, z.B. Ammoniumhydrogencarbonat.

Das erfindungsgemäße Polypeptid, dessen N-Terminus als freie Base vorliegt, kann in an sich bekannter Weise in das entsprechende N-Acyl oder N-Acylglycyl-Derivat überführt werden, wobei Acyl=BOC oder $C_1$—$C_6$-Alkanoyl, insbesondere Acetyl bevorzugt ist.

Unter pharmakologisch unbedenklichen Salzen des erfindungsgemäßen Peptids werden vor allem Alkali- und Erdalkalisalze, aber auch physiologisch annehmbare Salze mit Aminen, wie Alkyl- und Cycloalkylaminen verstanden.

Das erfindungsgemäße Peptid wurde in verschiedenen üblichen in vitro Tests hinsichtlich einer das Immunsystem beeinflussenden Wirkung als aktiv gefunden. Dazu gehört die T-Zell-Differenzierung, deren Ausmaß mit dem E-Rosetten-Test (Aiuti, Scand. J. Immunol., *3* 521—532 1974) und der Phythämagglutinin-Stimulationstest (PHA-Stimulationstest) gemessen wird.

In dem Spontanrosettentest wird die Reifung von Lymphozyten aus humanem Nabelschnurblut durch Rosettierung mit Schafserythrozyten in der Kälte erfolgt. Da allein reife T-Zellen in der Kälte mit Schafserythrozyten Rosetten bilden, ist dies ein spezifischer T-Zelltest, d.h. er gestattet die Beobachtung der Verschiebung von T-Zellen (sog. Nullzellen, die im Nabelschnurblut in relativ großer Zahl vorkommen) zur $R^+$-T-Zellen, d.h. reifen T-Zellen.

Der Normalwert an rosettenbildenden Zellen im Nabenschnurblut liegt wegen der größeren Anzahl an Nullzellen niedriger als im Peripherblut von Erwachsenen. Auf Grund von Untersuchungen des Blutes von Patienten, die an Autoimmunkrankheiten oder Tumoren erkrankt sind, läßt sich eine niedrige Rosettenzahl korrelieren mit einem niedrigen Immunstatus. Im PHA-Stimulationstest (PHA=Phythämagglutinin) oder auch Lymphozytentransformationstest werden Rückschlüsse über die Anzahl der reifen, d.h. stimulierbaren Lymphozyten nicht über eine Untersuchung der Oberflächenstrukturen gewonnen wie beim Spontanrosettentest, sondern durch den Funktionstest auf Stimulierbarkeit mit dem Pflanzenlectin PHA, das vor allem an reifen T-Zellen bindet.

Die T-Lymphozyten werden durch das Lectin, ähnlich wie durch bakterielle oder viralen Antigene zu einer Blastentransformation angeregt. Diese führt entweder direkt oder durch Ausschüttung von Lymphokinen zu einer Proliferation. Der Einbau von radioaktivem Thymidin innerhalb einer bestimmten Zeit ist dann ein Maß für die Anzahl der stimulierten Zellen. Da nur die reifen oder immunologisch potenten Lymphozyten stimuliert werden, läßt sich mit diesem Test die Reifung von aus Nabelschnurblut präparierten Lymphozyten nach Inkubation mit Substanz verfolgen.

Das Polypeptid wurde auch bei Versuchen mit Lymphozyten von immundefizienten Patienten, z.B. Die George Syndrom, in Bezug auf eine Erhöhung der T-Lymphozyten-Spiegel als besonders wirksam erkannt. Dabei wurde auch der gewünschte höhere Effekt, der sich in einer wesentlich geringeren Wirkkonzentration ausdrückt, für die reine Substanz gegenüber dem Gemisch entsprechend "Fraktion 5" klar ersichtlich (siehe Tabelle 1).

TABELLE 1

| Dosis µg/ml | "Fraktion 5" | | | erfindungsgemäßes Hormon | | |
|---|---|---|---|---|---|---|
| | 125 | 250 | 500 | 5 | 10 | 100 |
| Aktivität* | 311 | 244 | 111 | 133 | 322 | 88 |

* Konzentration von T-Lymphozyten in % gegenüber Standard nach Inkubation mit Fraktion 5 oder dem erfindungsgemäßen Polypeptid.

Die Erfindung betrifft auch die Verwendung des erfindungsgemäßen Peptids und seiner Derivate zur Regulierung oder Stimulierung des Immunsystems als Therapeutikum oder prophylaktisches Agens.

Die Erfindung betrifft weiterhin Arzneimittel mit immunstimulierender bzw. -regulierender Wirkung, die zur Behandlung und Vorbeugung von Virus-Infektionen, Immunmangel-Erkrankungen, Tumorbildung (besonders Krebs-Erkrankungen) und Formen beschleunigter Alterungsprozesse eingesetzt werden können und das erfindungsgemäße Polypeptid und/oder seine N-Acyl-Derivate in freier Form oder in Form pharmakologisch annehmbarer Salze enthalten. Bei der Therapie maligener Tumoren können die erfindungsgemäßen Peptide auch in Kombination mit Zytostatika (siehe z.B. Mutschler, Arzneimittelwirkungen, Wissenschaftliche Verlagsgesellschaft mbH Stuttgart 1981, Seite 599—608) und/oder Bestrahlungsmaßnahmen eingesetzt werden.

Die Anwendung der erfindungsgemäßen Polypeptide kann parenteral (z.B. subkutan oder intravenös) oder über die Schleimhäute (z.B. nasal oder enteral) erfolgen.

Die Dosierung bei parenteraler Gabe liegt, abhängig von der Art der Erkrankung, bei 0,01—50 mg/m² Körperoberfläche/Tag. Sie kann in schweren Fällen auch erhöht werden, da toxische Eigenschaften bisher nicht beobachtet wurden. In besonders schweren Fällen empfiehlt sich eine mehrfache Gabe pro Woche über einen mehrwöchigen Zeitraum. Bei nasaler Applikation muß, bedingt durch die besonderen Resorptionsverhältnisse, entsprechend höher dosiert werden.

Zur subkutanen, intravenösen oder nasalen Applikation werden die erfindungsgemäßen Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glukose- oder Mannitlösungen, oder auch eine Mischung aus den verschiedenen genannten Lösungsmitteln.

Die parenterale Applikation kann beispielsweise auch mit externer oder implantierten Dosiervorrichtungen, wie beispielsweise automatischen Pumpen, oder in Form einer Dauerinfusion erfolgen.

Hierbei ist es vorteilhaft, gegen Denaturierung stabilisierende Zusätze, wie sie beispielsweise aus der EP—A 18 609 bekannt sind, zuzusetzen.

Die erfindungsgemäßen Verbindungen können auch in Form eines Arzneimittels mit Depotwirkung appliziert werden. Bei Injektionslösungen kann eine solche Depotwirkung z.B. durch Lösung oder Suspendierung des Arzneistoffs in einem öligen Vehikel, Zugabe von viskositätserhöhenden Makromolekülen, welche die Diffusion der gelösten Arzneistoffe verzögern, Adsorption des Arzneistoffs an geeignete Trägermoleküle, z.B. Aluminiumhydroxid, oder Verwendung von Kristallsuspensionen erreicht werden.

Für eine orale Anwedungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsform gebracht, wie Tabletten, Dragees, Steckkapseln, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose oder Stärke, inbesonder Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tiereische Öle in Betracht, wie Sonnenblumenöl oder Lebertran.

Eine wäßrige sterile Lösung des erfindungsgemäßen Polypeptids und seiner Derivate kann Isotoniemittel wie z.B. etwa 0,9% Natriumchlorid oder 1,7% Glycerin und gegebenenfalls auch Konservierungsmittel wie Hydroxybenzoesäure oder Phenol enthalten.

Beispiel 1

500 g gefrorene Thymusdrüsen werden in einem Mixer mit 1,5 l eiskalter 0,09 %-iger Natriumchloridlösung und 5 ml Octanol innerhalb von 3 Minuten homogenisiert. Nach 30-minütiger Zentrifugation bei 0°C und 10 000 UpM wird der noch trübe Überstand mittels Filtration über 2 Lagen Gaze weiter geklärt und anschließend unter Rühren innerhalb von 15 Minuten auf 80°C erhitzt. Die entstandene Fällung wird durch Filtration über 4 Lagen Gaze abgetrennt. Das Filtrat wird durch Rühren in einem Eisbad schnell auf +4°C heruntergekühlt und in 7,5 l auf −20°C vorgekühltes Aceton einfließen gelassen. Es entstehet erneut eine Fällung, die nach 5 Minuten auf einer Glasfilternutsche abfiltriert und mit 1 l kaltem Aceton (−20°C) nachgewaschen wird. Nach Trocknung im Vakuum resultieren 6,1 g eines leicht gelblichen Pulvers mit einem Proteingehalt (bestimmt nach der Methode von Lowry) von 32%.

Beispiel 2

25 kg Thymus werden im gefrorenen Zustand (Anfangstemperatur −30°C) in einem Walzenbrecher vorzerkleinert und im Fleischwolf zermahlen (insgesamt 15 Minuten) und anschließend mit 75 l 0,9 %iger Natriumchloridlösung, hergestellt aus 74,3 kg pyrogenfreiem Wasser, 675 g Natriumchlorid und 0,75 g Phenylmethylsulfonylfluorid, im 100 l-Spritzkessel bei 0—4°C 5 Minuten homogenisiert. Über eine auf 0—4°C gekühlte Röhrenzentrifuge (Cepa®) werden innerhalb einer Stunde bei 15 000×g 3 Rotorfüllungen Feststoff (15 kg Feuchtgewicht) abgetrennt und verworfen. 83,6 kg Überstand werden über Gaze filtriert, das Filtrat unter Rühren innerhalb von 20—25 Minuten in einem 100 l-Spritzkessel auf +80°C Innentemperatur erwärmt und der entstandene Niederschlag (3 kg Feuchtgewicht) innerhalb von 15 Minuten mittels einer gekühlten Röhrenzentrifuge bei 15 000×g abgetrennt. Der leicht opaleszierende Überstand wird über 4 Lagen Gaze filtriert und unter Rühren in 375 l auf −20°C vorgekühltes Aceton gegeben. Nach 2 Minuten wird der entstandene Niederschlag über eine gekühlte Röhrenzentrifuge abgeschleudert (15 000×g, 15 Minuten). Der Zentrifugenrückstand (500 g Feuchtgewicht) wird mit 3 l Aceton von −20°C gewaschen, in einer Becherzentrifuge bei 3 000×g in 10 Minuten abgeschleudert und im Vakuum-Exsikkator getrocknet. Es resultieren 325 g eines leicht gelblichen Pulvers mit einem Proteingehalt von 25%.

Beispiel 3

250 g Acetonpulver gemäß Beispiel 1 bzw. 2 werden in 2,5 l Wasser gelöst, mit 5 N Ammoniumhydroxyd auf pH 8,0 gestellt und eine Stunde bei 0—+4°C gerührt. Der unlösliche Anteil (etwa 50 g nach Trocknung) wird innerhalb von 30 Minuten mit einer Becher-Zentrifuge mit 5000 UpM abgeschleudert. Im Überstand wird nach Bestimmung der Proteinmenge mit der üblicherweise angewendeten Methode nach Lowry durch Zugabe von 0,7 l Wasser ein Proteingehalt von etwa 25 mg/ml eingestellt, die Lösung mit 1 l gesättigter Ammoniumsulfat-Lösung versetzt und 1 Stunde bei +4°C gerührt. Es entsteht ein Niederschlag, der durch Zentrifugation (5000 UpM, 30 min.) abgetrennt wird. Der klare Überstand wird mit 628 g Ammoniumsulfat versetzt und mit Eisessig auf pH 4,0 eingestellt. Nach einstündigem Rühren bei 0—+4°C wird die entstandene Suspension wiederum einer Zentrifugation unterworfen. Der dabei gewonnene Niederschlag enthält etwa 35 g Protein, bestimmt nach der Methode von Lowry, neben einer erheblichen Menge von Ammoniumsulfat und wird daher nicht getrocknet, sondern direkt für die Ultrafiltration eingesetzt.

Beispiel 4

Die aus 250 g Acetonpulver erhaltene Ammoniumsulfatfällung gemäß Beispiel 3 wird in 1,5 l 0,1 N Ammoniumhydrogencarbonat (pH 8,0) klar gelöst und in einer Amicon-Hollow-Fiber Apparatur mit 2 Filterpatronen des Typs H 10×50 (Ausschlußgrenze 50 000 Dalton) einer Ultrafiltration unterworfen. Dabei wird die Lösung bis auf 300 ml aufkonzentriert, wobei gegen Ende der Ultrafiltration zweimal je 500 ml 0,1 N Ammoniumhydrogencarbonat (pH 8,0) nachgefüllt werden, um eine vollständige Abtrennung aller Proteine unter 50 000 Dalton zu erreichen. Der alle höhermolekularen Proteine enthaltende Rückstand fällt nach Gefriertrocknung in einer Menge von 19,8 g mit einem Proteingehalt nach Lowry in Höhe 84% an.

Das Filtrat wird einer erneuten Ultrafiltration, diesmal in einer 2,5 l Amicon-Zelle mit einer 5PM10-Flachmembran (Ausschlußgrenze 10 000 Dalton), unterzogen. Dabei wird die Lösung auf 150 ml eingeengt, wobei gegen Ende zweimal 400 ml 0,1 N Ammoniumhydrogencarbonat (pH 8,0) nachgefüllt werden. Gefriertrocknung des Rückstandes ergibt 8,2 g mit einem Proteingehalt von 89%.

Zur Befreiung des Filtrats von Salzen, vor allem Ammoniumsulfat, wird eine Säule mit 21,5 cm Durchmesser und 100 cm Länge verwendet, die mit Sephadex G10 (coarse) gefüllt und mit 0,1 M Ammoniumhydrogencarbonat (pH 8,0) äquilibriert wurde. Sie wird mit einem Durchfluß von 5 l pro Stunde betrieben. Nach etwa einem Drittel des Säulenvolumens erscheint im Eluat salzfreies Protein, gefolgt von einer Mischfraktion aus Protein und Salz (Proteingehalt nach Lowry 48%), die einer Rechromatographie über die gleiche Säule unterworfen wird. Auf diese Weise erhält man, nach Gefriertrocknung, 5,9 g eines Materials mit einem Proteingehalt von 93%.

Beispiel 5

17 g einer durch Ultrafiltration gemäß Beispiel 4 erhaltenen Proteinfraktion werden mit 500 ml 0,01 M Natriumacetatpuffer (pH 5,5) verrührt. Ein unlöslicher Rückstand (1,15 g nach Trocknung) wird durch Zentrifugation (20 Minuten, 5000 UpM) abgetrennt. Der lösliche Anteil wird mit 3 l CM-Cellulose (Servacel CM-23), die mit dem gleichen Puffer äquilibriert wurde, versetzt. Nach halbstündigem Rühren wird die Suspension über eine Glasfilternutsche gegeben, der Kationenaustauscher bis fast zur Trockenheit abgesaugt und mit 2 l des gleichen Puffers langsam nachgewaschen.

Das Filtrat (2,5 l) wird gefriergetrocknet, in 150 ml 0,1 M Ammoniumhydrogencarbonat (pH 8,0) gelöst und über eine Säule mit 5 cm Durchmesser und 40 cm Länge, die mit Sephadex G10 (coarse) gefüllt und mit 0,1 M Ammoniumhydrogencarbonat (pH 8,0) äquilibriert wurde, bei einem Durchfluß von 150 ml pro Stunde entsalzt. Auch hier erhält man zunächst eine Fraktion mit salzfreiem Protein gefolgt von einer Mischfraktion aus Protein und Salz, die nochmals über die gleiche Säule rechromatographiert wird. Man erhält so, nach Gefriertrocknung, 9,1 g eines Materials mit einem Proteingehalt von 94%, das im folgenden als Fraktion bezeichnet wird.

Beispiel 6

30 g einer durch Ultrafiltration gemäß Beispiel 4 erhaltenen Proteinfraktion werden in 1 l 0,01 M Natriumacetat (pH 5,5) eingerührt und ein unlöslicher Anteil (1,3 g nach Trocknung) durch Zentrifugation (20 Minuten, 5000 UpM) abgetrennt.

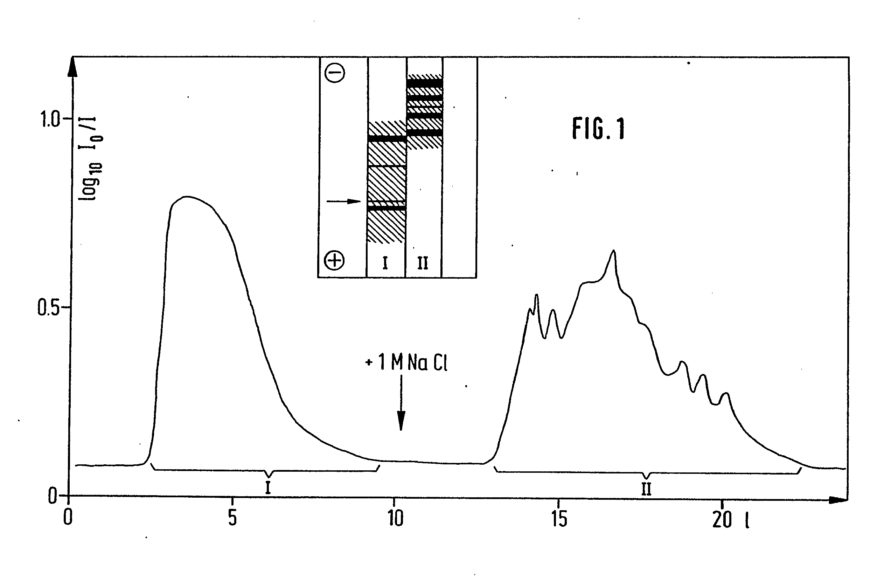

Die klare Lösung wird einer Chromatographie über eine Säule mit 10 cm Durchmesser und 43 cm Länge, gefüllt mit CM-Cellulose (Servacel CM-32), die mit 10 l 0,01 M Natriumacetat (pH 5,5) äquilibriert wurde, unterworfen, wobei zunächst nach Probeauftrag bei einer Flußgeschwindigkeit von 500 ml pro Stunde mit 10 l des gleichen Puffers eluiert wird. Dabei erhält man einen Peak (s. Abb. 1), der wie in Beispiel 5 beschrieben von Salzteilen befreit wird. Es resultieren aus der Gefriertrocknung 17,5 g einer Fraktion (in der Folge als "CM1' bezeichnet) mit einem Proteingehalt von 95%.

Durch Pufferwechsel auf 0,01 M Natriumacetat, 1,0 M Natriumchlorid (pH 5,5) wird die Säule für eine erneute Verwendung freigewaschen. Dabei erhält man einen weiteren Peak (s. Abb. 1), der nach Entsalzung in der üblichen Weise und Gefriertrocknung 11,5 g Protein liefert.

Beispiel 7

Eine Säule mit 10 cm Durchmesser und 62 cm Länge wird mit DEAE-Sephacel® (Pharmacia), das in einem Puffer aus 0,05 M Tris/HCl (Trishydroxymethyl-aminomethan) (pH 8,5) vorgequollen worden ist, gefüllt und bei einer Durchflußrate von 325 ml pro Stunde mit 15 l des gleichen Puffers äquilibriert.

17,5 g CM1 werden in 1 l 0,05 M Tris/HCl (pH 8,5) gelöst und mit 325 ml pro Stunde auf die Säule gepumpt. Mit der gleichen Geschwindigkeit wird mit 1 l des gleichen Puffers nachgespült und schließlich ein linearere Gradient von 0—1,0 M Natriumchlorid über ein Volumen von 36 l angelegt. Durch Detektion bei 245 nm wird eine Vielzahl kleiner Peaks sichtbar (s. Abb. 2), die sich zu einem großen Hauptpeak überlagern. Jeder dieser Peaks wird in der üblichen Weise entsalzt und durch basische Polyacrylamidgel-Elektrophorese (PAGE) analysiert. Danach befindet sich die gesuchte Substanz mit einem $R_F$-wert von 0,5—0,6 im dritten Gipfel des Hauptpeaks (in Abb. 2 mit 2 gekennzeichnet), der 1158 mg Protein enthält.

Vergleich der PAGE des die Substanz enthaltenden Gipfels und der davor eluierten Fraktion (s. Einschub in Abb. 1) zeigt die in diesem Stadium der Reinigung kaum vermeidbare Überlappung der einzelnen Peaks, die eine Nachreinigung der einzelnen Peaks unumgänglich macht.

7

Beispiel 8

Eine Säule mit 2,6 cm Durchmesser und 25 cm Länge wird mit in 0,01 M Natriumphosphatpuffer (pH 6,8) aufgeschwämmtem Hydroxylapatit (Bio Rad) gefüllt und bei einer Durchflußrate von 80 ml pro Stunde mit 500 ml des gleichen Puffers äquilibriert.

400 mg der die gesuchte Substanz enthaltenden Fraktion aus der Anionenaustauschchromatographie werden in 50 ml 0,01 M Natriumphosphatpuffer (pH 6,8) gelöst und mit 80 ml des gleichen Puffers wird ein linearer Gradient von 0,01—0,40 M Natriumphosphat (pH 6,8) über 1,3 l angelegt (s. Abb. 3). Die gesuchte Substanz befindet sich im Druchbruchspeak (Peak 1 in Abb. 3) und wird aus diesem zusammen mit einigen anderen Polypeptiden durch in der üblichen Weise durchgeführte Entsalzung und Gefriertrocknung in einer Ausbeute von 290 mg isoliert.

Das erhaltene Material zeigt in der PAGE ein ähnliches Bandenspektrum wie das Ausgangsgemisch, aber mit erheblich stärkerer Intensität der dem erfindungsgemäßen Polypeptid entsprechenden Bande.

Beispiel 9

Eine Säule mit 1,6 cm Durchmesser und 120 cm Länge wird mit in 0,1 M Ammoniumhydrogencarbonatpuffer (pH 8,0) vorgequollenem Ultrogel ACA 54 (LKB) gefüllt und bei einer Durchflußgeschwindigkeit von 30 ml pro Stunde mit 750 ml des gleichen Puffers äquilibriert.

290 mg der die gesuchte Substanz enthaltenden Fraktion aus der Anionenaustauschchromatographie werden in 30 ml 0,1 M Ammoniumhydrogencarbonatpuffer (pH 8,0) gelöst und mit 30 ml pro Stunde auf die Säule gepumpt, die anschließend mit dem gleichen Puffer bei gleicher Durchflußgeschwindigkeit weitergespült wird (s. Abb. 4).

Es erscheint ein kleiner Durchbruchspeak, der geringe Mengen hochmolekularer Proteine enthält, und nach dem doppelten Volumen ein breiter Hauptpeak, der über das gesamte Volumen hinweg das gleiche Bandenspektrum in der PAGE liefert.

Gefriertrocknung dieses Hauptpeaks ergibt 200 mg eines Materials, das in seiner Qualität gegenüber dem Ausgangsmaterial nur geringfügig verbessert ist.

Beispiel 10

Eine Säule mit 2,5 cm Durchmesser und 35 cm Länge wird mit DEAE-Sephacel (Pharmacia), vorgequollen in 0,005 M Tris/HCl (pH 8,0) gefüllt und bei einer Durchflußgeschwindigkeit von 60 ml pro Stunde mit 500 ml des gleichen Puffers äquilibriert.

220 mg der die gleiche Substanz enthaltenden Hydroxylapatit-Fraktion werden in 50 ml 0,05 M Tris/HCl (pH 8,0) gelöst und mit 60 ml pro Stunde auf diese Säule gepumpt. Anschließend wird bei der gleichen Geschwindigkeit mit einem linearen Gradienten von 0 bis 0,3 M Natriumchlorid über ein Volumen von 2 l eluiert (s. Abb. 5).

Es erscheint etwa in der Mitte des Gradienten ein aus mehreren Gipfeln zusammengesetzter Hauptpeak. Trennt man diesen in 5 Fraktionen auf (s. Abb. 5), so befindet sich die erfindungsgemäße Substanz überwiegend in Fraktion 4. Aus dieser kann sie durch in der üblichen Weise ausgeführte Entsalzung und Gefriertrocknung in einer Ausbeute von 70 mg und mit einer Reinheit von über 90% laut PAGE isoliert werden.

Beispiel 11: Typische Spaltung

Bei der enzymatischen Spaltung wurde mit einem Enzym-Substrat-Verhältnis von 1/100 bei einer Konzentration von 1 mg/ml Substrat in 0,1 M N-Methylmorpholin-Acetat-Puffer pH 8,1 gearbeitet. Es wurde 2 Stunden mit TPCK behandeltem Trypsin oder 7 Stunden mit TLCK behandeltem α-Chymotrypsin bei 37°C inkubiert. Nach Ansäuern auf pH 2,5 mit 6 N Eisessig wurde die Lösung schnell auf 80°C erhitzt. Nach 2 Minuten wurde wieder rasch abgekühlt und zentrifugiert. Der Überstand wurde anschließend lyophylisiert, wobei ein salzfreies, weißes Pulver anfiel.

Beispiel 12: E-Rosetten Test

Im Test, der nach der Methode von Aiuti (Scand. J. Immunol., 3 521—532 1974) durchgeführt wird, werden die Rosettenzahlen von Lymphozyten-Erythrozyten-Systemen nach zweistündiger Inkubation (37°C) in Medium bzw. in Medium+Substanz bestimmt. In der Auswertung werden von 200 gezählten Zellen nur die als Rosetten gewertet, die mindestens aus einem Lymphozyt und drei Erythrozyten besteht.

Der Quotient aus der Rosettenzahl nach Vorinkubation mit Substanz und ohne Substanz, der Stimulationsindex $S_i$, ist dann ein Maß für die durch die Substanz ausgelöst Differenzierung von Nullzellen zu reifen T-Zellen.

Ergebnisse:

| Dois µg/ml | Thymosin Fraktion V | erfindungsgemäße Polypeptid |
|---|---|---|
| 20 | $S_I=1,9$ | $S_I=3,1$ |
| 10 | 2,6 | / |
| 5 | 2,0 | 2,5 |
| 2,5 | 1,9 | / |
| 1,25 | 1,3 | 2,7 |

Während die Fraktion V als Gemisch verschiedener Peptide unterschiedlicher biologischer Aktivität bei einer Konzentration von 1,25 µg/ml nur eine Erhöhung der Rosettenzahl von 17% auf 22% bewirkt, führt eine Inkubation der Lymphozyten mit 1,25 µg/ml des erfindungsgemäßen Peptids zu einer Erhöhung der Rosettenzahl von 18,5% auf 50,5%.

Beispiel 12a: Phythämagglutinin-Stimulationstest

Die Substanz(en) werden in verschiedenen Konzentrationen zu der Zellsuspension gegeben ($10^5$ Zellen in 100 µl RPMI 1640/Click+Hepes 20 mM, Penicillin; Streptomycin Glutamin und 10% AB-Serum hitzeinaktiviert) und entweder nach 48 h oder nach 72 h etwa 1 µCi/Bohrung Tritium markiertes Thymidin zugegeben, 8 h inkubiert, die Zellen abgesaugt auf Glasfaserfilter, gewaschen und die Filter später in Szintillationsflüssigkeit gegeben und im β-Counter gezählt.

Da die Substanz(en) während der ganzen Kulturdauer präsent ist, kann ein möglicher Abbau bzw. Veränderung durch Serum- oder Lymphozytenproteasen nicht ausgeschlossen werden.

Ergebnisse:

| Substanz | Konzentration PHA (µg/ml) | Inkubation Zeit (Std.) | Dosis (µg) | cpm | $x_1$ | Kontrolle | $\bar{x}_2$ | $S=\dfrac{x^1}{x_2}$ |
|---|---|---|---|---|---|---|---|---|
| erfindungs- gemäße Polypeptid | 10 | 48 | 1 | 24772 20334 17791 | 20965 | 16 216 12 730 | 14472 | 1,45 |

Erläuterungen zu den Abbildungen.

Figur 1
Fraktionierung des durch Ultrafiltration erhaltenen Materials an CM-Cellulose
10×43 cm Servacel CM 32 in 0,1 M Natriumacetat, pH 5,5 Flußgeschw.: 500 ml/h
Aufgabemenge: 30 g
Nach 10 l: Pufferwechsel (jetzt 0,1 Natriumacetat, 1 M Natriumchlorid, pH 5,5).
Der Einschub zeigt das Bild der basischen Polyacrylamidgelelektrophorese.

Ausbeuten:
Fraktion I : 17,5 g
Fraktion II: 11,5 g.

Figur 2
Auftrennung der obengenannten Fraktion I von CM-Cellulose an DEAE-Cellulose
10×62 cm DEAE-Sephacel in 0,05 M Tris/HCl, pH 8,5 Flußgeschw.: 350 ml/h
Gradient von 0—1 M Natriumchlorid über 36 l
Aufgabemenge: 17,5 g.
Der Einschub zeigt das Bild der basischen Polyacrylamidgelelektrophorese der das erfindungsgemäße Polypeptid enthaltenden und der benachbarten Fraktion.

Ausbeuten:
Fraktion I : 722 mg
Fraktion II: 1158 mg.

Figur 3
Reinigung einer DEAE-Cellulose-Fraktion an Hydroxylapatit
    2,6×25 cm Hydroxylapatit in 0,01 M Natriumphosphat, pH 6,8
    Flußgeschw.: 80 ml/h
    Gradient von 0,01—0,40 M
    Phosphat, pH 6,8 über 1,3 l
    Aufgabemenge: 400 mg.
    Der Einschub zeigt das Bild der basischen Polyacrylamidgelelektrophorese.

Ausbeuten:
    Fraktion I : 290 mg
    Fraktion II : 40 mg
    Fraktion III: 9 mg.

Figur 4
Gelfiltration einer DEAE-Cellulose-Fraktion
    1,6×120 cm Ultrogel ACA 54 in 0,1 M
    Ammoniumhydrogencarbonat, pH 8,0
    Flußgeschw.: 30 ml/h
    Aufgabemenge: 290 mg.
    Der Einschub zeigt das Bild der basischen Polyacrylamidgelelektrophorese.

Ausbeuten:
    Fraktion I : 2 mg
    Fraktion II : 72 mg
    Fraktion III: 128 mg.

Figur 5
Rechromatographie der Hydroxylapatit-Fraktion 1 über DEAE-Cellulose
    2,5×35 cm DEAE-Sephacel in 0,05 M Tris/HCl, pH 8,0
    Flußgeschw.: 60 ml/h
    Gradient von 0—0,3 M Natriumchlorid über 2 l
    Aufgabemenge: 220 mg.
    Der Einschuß ziegt das Bild der basischen Polyacrylamidgelelektrophorese.

Ausbeuten:
    Fraktion I : 28 mg
    Fraktion II : 53 mg
    Fraktion III: 42 mg
    Fraktion IV: 70 mg
    Fraktion V : 12 mg.

**Patentansprüche für die Vertragsstaaten: BE, CH, DE, FR, GB, IT, LI, LU, NL, SE**

1. Polypeptid mit Wirkung auf das Immunsystem, gekennzeichnet durch ein Molekulargewicht von 3480, durch eine UV-Absorption mit einem Maximum bei 205 bis 210 nm, durch einen isoelektrischen Punkt von 3,95±0,15 und durch folgende Aminosäurezusammensetzung:
4 Asparaginsäure, 3 Threonin, 3 Serin, 6 Glutaminsäure, 1 Glycin, 4 Alanin, 3 Valin, 1 Isoleucin, 1 Leucin, 5 Lysin, 1 Arginin,
wobei der N-Terminus gegebenenfalls eine Acylgruppe von 1 bis 6 C-Atomen oder eine Acylglycylgruppe, deren Acrylgruppe 1 bis 6 Kohlenstoffatome aufweist, trägt, sowie dessen pharmakologisch unbedenklichen Salze.

2. Verfahren zur Gewinnung eines gereinigten Polypeptids gemäß Anspruch 1, dadurch gekennzeichnet, daß man das Polypeptid aus Thymus mit Hilfe einer Kombination von Fällungsmethoden, Membranfiltrationen und Chromatographie isoliert und das erhaltene Peptid gegebenenfalls in an sich bekannter Weise in sein N-Acyl- oder N-Acylglycylderivat überführt und gegebenenfalls in ein Salz überführt.

3. Verfahren nach Anspruch 2, gekennzeichnet durch Extraktion der Drüsen mit einer wäßrigen Lösung eines anorganischen Salzes mit puffernder Wirkung im pH Bereich von 5,0 bis 9,0, Abtrennen des unlöslichen Materials und Isolierung des Polypeptids aus dem so erhaltenen Extrakt mit an sich bekannten Trenenverfahren.

4. Verfahren nach Anspruch 2, gekennzeichnet dadurch, daß der chromatographische Schritt aus einer Kationen- und/oder Anionen-Austauschchromatographie, einer Chromatographie an unspezifischen Adsorbentien, einer Verteilungschromatographie, oder einer Kombination aus zwei oder mehr dieser Methoden besteht.

10

5. Verfahren nach Anspruch 2, gekennzeichnet dadurch, daß man für die Chromatographie gemäß Anspruch 4 einen Extrakt einsetzt, den man einer Hitzedenaturierung unterzogen hat, wobei das Peptid nach Anspruch 1 gelöst bleibt, während sich andere Proteine durch Sedimentation oder Filtration abtrennen lassen.

6. Verfahren nach Anspruch 4, gekennzeichnet dadurch, daß man für die Chromatographie ein Material einsetzt, das bei der Behandlung des Extrakts gemäß Anspruch 3 und/oder 5 durch Zugabe von organischen Lösungsmitteln, die mit Wasser mischbar sind, als Niederschlag abgetrennt wird.

7. Verfahren nach Anspruch 4 gekennzeichnet dadurch, daß man für die Chromatographie einen Extrakt einsetzt, den man einer fraktionierten Fällung durch Zugabe wasserlöslicher anorganischer und/oder organischer Salze unterzogen hat, wobei das Peptid in bestimmten, vom verwendeten Salz abhängigen Konzentrationsbereichen ausfällt und durch Sedimentationstechniken angereichert wird.

8. Verfahren nach Anspruch 4, gekennzeichnet dadurch, daß man für die Chromatographie einen Extrakt einsetzt, der durch Fraktionierung mittels Membranfiltration und/oder Gelfiltration nur noch Peptide enthält, die kleiner sind als 10 000 Dalton.

9. Verfahren nach Anspruch 4, gekennzeichnet dadurch, daß man für die Chromatographie einen Extrakt einsetzt, den man aus der Kombination von zwei oder mehr Verfahren nach den Ansprüchen 5—8 erhalten hat.

10. Verwendung des Peptids nach Anspruch 1 als Therapeutikum oder prophylaktisches Agens zur Regulierung oder Stimulierung des Immunsystems.

11. Arzneimittel zur Regulierung oder Stimulierung des Immunsystems gekennzeichnet durch den Gehalt wirksamer Mengen des Polypeptids gemäß Anspruch 1 in freier Form oder in Form eines pharmakologisch annehmbaren Salzes.

12. Wäßrige sterile Lösung eines gereinigten Polypeptids gemäß Anspruch 1 in freier Form oder in Form eines pharmakologisch annehmbaren Salzes, enthaltend ein isotoniemittel und gegebenenfalls ein Konservierungsmittel.

13. Peptid gemäß Anspruch 1 zur Verwendung als Heilmittel.

**Patentansprüche für den Vertragsstaat: AT**

1. Verfahren zur Gewinnung eines gereinigten Polypeptids mit Wirkung auf das Immunsystem mit einem Molekulargewicht von 3480, einer UV-Absorption mit einem Maximum bei 205 bis 210 nm, einem isoelektrischen Punkt von 3,95±0,15 und folgender Aminosäurezusammensetzung:

4 Asparaginsäure, 3 Threonin, 3 Serin, 6 Glutaminsäure, 1 Glycin, 4 Alanin, 3 Valin, 1 Isoleucin, 1 Leucin, 5 Lysin, 1 Arginin,

wobei der N-Terminus gegebenenfalls eine Acylgruppe von 1 bis 6 C-Atomen oder eine Acylglycylgruppe, deren Acrylgruppe 1 bis 6 Kohlenstoffatome aufweist, trägt, sowie dessen pharmakologisch unbedenklichen Salzen, dadurch gekennzeichnet, daß man das Polypeptid aus Thymus mit Hilfe einer Kombination von Fällungsmethoden, Membranfiltrationen und Chromatographie isoliert und das erhaltene Peptid gegebenenfalls in an sich bekannter Weise in sein N-Acyl- oder N-Acylglycylderivat überführt und gegebenenfalls in ein Salz überführt.

2. Verfahren nach Anspruch 1, gekennzeichnet durch Extraktion der Drüsen mit einer wäßrigen Lösung eines anorganischen Salzes mit puffernder Wirkung im pH-Bereich von 5,0 bis 9,0, Abtrennen des unlöslichen Materials und Isolierung des Polypeptids aus dem so erhaltenen Extrakt mit an sich bekannten Trennverfahren.

3. Verfahren nach Anspruch 1, gekennzeichnet dadurch, daß der chromatographische Schritt aus einer Kationen- und/oder Anionen-Austauschchromatographie, einer Chromatographie an unspezifischen Adsorbentien, einer Verteilungschromatographie, oder einer Kombination aus zwei oder mehr dieser Methoden besteht.

4. Verfahren nach Anspruch 1, gekennzeichnet dadurch, daß man für die Chromatographie gemäß Anspruch 3 einen Extrakt einsetzt, den man einer Hitzedenaturierung unterzogen hat, wobei das Peptid nach Anspruch 1 gelöst bleibt, während sich andere Proteine durch Sedimentation oder Filtration abtrennen lassen.

5. Verfahren nach Anspruch 3, gekennzeichnet dadurch, daß man für die Chromatographie ein Material einsetzt, das bei der Behandlung des Extrakts gemäß Anspruch 2 und/oder 4 durch Zugabe von organischen Lösungsmitteln, die mit Wasser mischbar sind, als Niederschlag abgetrennt wird.

6. Verfahren nach Anspruch 3 gekennzeichnet dadurch, daß man für die Chromatographie einen Extrakt einsetzt, den man einer fraktionierten Fällung durch Zugabe wasserlöslicher anorganischer und/oder organischer Salze unterzogen hat, wobei das Peptid in bestimmten, vom verwendeten Salz abhängigen Konzentrationsbereichen ausfällt und durch Sedimentationstechniken angereichert wird.

7. Verfahren nach Anspruch 3, gekennzeichnet, dadurch daß man für die Chromatographie einen Extrakt einsetzt, der durch Fraktionierung mittels Membranfiltration und/oder Gelfiltration nur noch Peptide enthält, die kleiner sind als 10 000 Dalton.

8. Verfahren nach Anspruch 3, gekennzeichnet dadurch, daß man für die Chromatographie einen Extrakt einsetzt, den man aus der Kombination von zwei oder mehr Verfahren nach den Ansprüchen 4 bis 7, erhalten hat.

9. Verfahren zur Gewinnung eines gereinigten Polypeptids gemäß einem der Ansprüche 1 bis 8, dadurch gekennzeichnet, daß das im Anspruch 1 definierte Peptid zur Verwendung als Heilmittel hergestellt wird.

10. Verfahren zur Herstellung eines Arzneimittels zur Regulierung oder Stimulierung des Immunsystems, dadurch gekennzeichnet, daß man eine wirksame Menge des Polypeptids gemäß Anspruch 1 in freier Form oder in Form eines pharmakologisch annehmbaren Salzes in eine geeignete Zubereitungsform bringt.

11. Verfahren zur Herstellung einer wäßrigen sterilen Lösung eines gereinigten Polypeptids gemäß Anspruch 1 in freier Form eines pharmakologisch annehmbaren Salzes, enthaltend ein Isotoniemittel und gegebenenfalls ein Konservierungsmittel, dadurch gekennzeichnet, daß man diese in Lösung bringt.

**Revendications pour les Etats Contractants: BE, CH, DE, FR, GB, IT, LI, LU, NL, SE**

1. Polypeptide à action sur le système immunitaire, caractérisé par une masse moléculaire de 3 480, par une absorption UV avec un maximum à 205—210 nm, par un point isoélectrique de 3,95±0,15 et par la composition suivante en aminoacides:

4 restes acide aspartique, 3 thréonine, 3 sérine, 6 acide glutamique, 1 glycine, 4 alanine, 3 valine, 1 isoleucine, 1 leucine, 5 lysine, 1 arginine,

l'extrémité N-terminale comportant éventuellement un groupe acide ayant de 1 à 6 atomes de carbone, ou un groupe acylglycyle dont le fragment acyle comporte de 1 à 6 atomes de carbone, et sels pharmacologiquement acceptables de celui-ci.

2. Procédé pour l'obtention d'un polypeptide purifié selon la revendication 1, caractérisé en ce que l'on isole le polypeptide à partir du thymus à l'aide d'une combinaison de méthodes de précipitation, de filtrations sur membrane et de chromatographie, et éventuellement on convertit d'une façon connue en soi le peptide obtenu en son dérivé N-acyle ou N-acylglycyle et éventuellement on le convertit en un sel.

3. Procédé selon la revendication 2, caractérisé par l'extraction des glandes à l'aide d'une solution aqueuse d'un sel minéral à effet tampon dans l'intervalle de pH de 5,0 à 9,0, séparation de la matière insoluble et isolement du polypeptide à partir de l'extrait ainsi obtenu, à l'aide d'un procédé de séparation connu.

4. Procédé selon la revendication 2, caractérisé en ce que l'étape de chromatographie consiste en une chromatographie d'échange de cations et/ou d'anions, une chromatographie sur adsorbants non spécifiques, une chromatographie de partage ou une combinaison de deux de ces méthodes ou plus.

5. Procédé selon la revendication 2, caractérisé en ce que, pour la chromatographie selon la revendication 4, on utilise un extrait que l'on soumet à une dénaturation à la chaleur, le peptide selon la revendication 1 restant en solution, tandis que d'autres protéines peuvent être séparées par sédimentation ou filtration.

6. Procédé selon la revendication 4, caractérisé en ce que, pour la chromatographie, on utilise un matériau qui est séparé sous forme de précipité lors du traitement de l'extrait selon la revendication 3 et/ou 5, par addition de solvants organiques qui sont miscibles à l'eau.

7. Procédé selon la revendication 4, caractérisé en ce que, pour la chromatographie, on utilise un extrait que l'on soumet à une précipitation fractionnée par addition de sels organiques et/ou minéraux solubles dans l'eau, le peptide précipitant dans certaines plages de concentration dépendant du sel utilisé et étant concentré par des techniques de sédimentation.

8. Procédé selon la revendication 4, caractérisé en ce que, pour la chromatographie, on utilise un extrait qui, grâce à un fractionnement par filtration sur membrane et/ou filtration sur gel, ne contient plus que des peptides qui ont une masse moléculaire inférieure à 10 000 daltons.

9. Procédé selon la revendication 4, caractérisé en ce que, pour la chromatographie, on utilise un extrait que l'on obtient par la combinaison de deux procédés ou plus selon les revendications 5 à 8.

10. Utilisation du peptide selon la revendication 1, en tant qu'agent thérapeutique ou agent prophylactique pour la régulation et/ou la stimulation du système immunitaire.

11. Médicament pour la régulation ou la stimulation du système immunitaire, caractérisé par la teneur en quantités efficaces du polypeptide selon la revendication 1, sous forme libre ou sous forme d'un sel pharmacologiquement acceptable.

12. Solution aqueuse stérile d'un polypeptide purifié selon la revendication 1, sous forme libre ou sous forme d'un sel pharmacologiquement acceptable, contenant un agent d'isotonie et éventuellement un conservateur.

13. Peptide selon la revendication 1, destiné à être utilisé comme médicament.

**Revendications pour l'Etat Contractant: AT**

1. Procédé pour l'obtention d'un polypeptide purifié à action sur le système immunitaire, caractérisé par une masse moléculaire de 3 480, par une absorption UV avec un maximum à 205—210 nm, par un point isoélectrique de 3,95±0,15 et par la composition suivante en aminoacides:

4 restes acide aspartique, 3 thréonine, 3 sérine, 6 acide glutamique, 1 glycine, 4 alanine, 3 valine, 1 isoleucine, 1 leucine, 5 lysine, 1 arginine,

l'extrémité N-terminale comportant éventuellement un groupe acide ayant de 1 à 6 atomes de carbone, ou un groupe acylglycyle dont le fragment acyle comporte de 1 à 6 atomes de carbone,

ainsi que de ses sels pharmacologiquement acceptables, caractérisé en ce que l'on isole le polypeptide à partir du thymus à l'aide d'une combinaison de méthodes de précipitation, de filtrations sur membrane et de chromatographie, et éventuellement on convertit d'une façon connue en soi le peptide obtenu en son dérivé N-acyle ou N-acylglycyle et éventuellement on le convertit en un sel.

2. Procédé selon la revendication 1, caractérisé par l'extraction des glandes à l'aide d'une solution aqueuse d'un sel minéral à effet tampon dans l'intervalle de pH de 5,0 à 9,0, séparation de la matière insoluble et isolement du polypeptide à partir de l'extrait ainsi obtenu, à l'aide d'un procédé de séparation connu.

3. Procédé selon la revendication 1, caractérisé en ce que l'étape de chromatographie consiste en une chromatographie d'échange de cations et/ou d'anions, une chromatographie sur adsorbants non spécifiques, une chromatographie de partage ou une combinaison de deux de ces méthodes ou plus.

4. Procédé selon la revendication 1, caractérisé en ce que, pour la chromatographie selon la revendication 3, on utilise un extrait que l'on soumet à une dénaturation à la chaleur le peptide selon la revendication 1 restant en solution, tandis que d'autres protéines peuvent être séparées par sédimentation ou filtration.

5. Procédé selon la revendication 3, caractérisé en ce que, pour la chromatographie, on utilise un matériau qui est séparé sous forme de précipité lors du traitement de l'extrait selon la revendication 2 et/ou 4, par addition de solvants organiques qui sont miscibles à l'eau.

6. Procédé selon la revendication 3, caractérisé en ce que, pour la chromatographie, on utilise un extrait que l'on soumet à une précipitation fractionnée par addition de sels organiques et/ou minéraux solubles dans l'eau, le peptide précipitant dans certaines plages de concentration dépendant du sel utilisé et étant concentré par des techniques de sédimentation.

7. Procédé selon la revendication 3, caractérisé en ce que, pour la chromatographie, on utilise un extrait qui, grâce à un fractionnement par filtration sur membrane et/ou filtration sur gel, ne contient plus que des peptides qui ont une masse moléculaire inférieure à 10 000 daltons.

8. Procédé selon la revendication 3, caractérisé en ce que, pour la chromatograhie, on utilise un extrait que l'on obtient par la combinaison de deux procédés ou plus selon les revendications 4 à 7.

9. Procédé pour l'obtention d'un polypeptide purifié selon l'une des revendications 1 à 8, caractérisé en ce que l'on prépare le peptide défini dans la revendication 1, pour l'utilisation en tant que médicament.

10. Procédé pour la préparation d'un médicament pour la régulation ou la stimulation du système immunitaire, caractérisé en ce que l'on met sous une forme pharmaceutique appropriée une quantité efficace du polypeptide selon la revendication 1, sous former libre ou sous forme d'un sel pharmacologiquement acceptable.

11. Procédé pour la préparation d'une solution aqueuse stérile d'un polypeptide purifié selon la revendication 1, sous forme libre ou sous forme d'un sel pharmacologiquement acceptable, contenant un agent d'isotonie et éventuellement un conservateur, caractérisé en ce que l'on met ce polypeptide en solution.

**Claims for the Contracting States: BE, CH, DE, FR, GB, IT, LI, LU, NL, SE**

1. A polypeptide having an action on the immunological system, which has a molecular weight of 3,480, a UV absorption with a maximum at 205 to 210 nm, an isoelectric point at 3.95±0.15 and the following aminoacid composition: 4 aspartic acid units, 3 threonine units, 3 serine units, 6 glutamic acid units, 1 glycine unit, 4 alanine units, 3 valine units, 1 isoleucine unit, 1 leucine unit, 5 lysine units and 1 arginine unit, the N-terminal, if appropriate, carrying an acyl group having 1 to 6 carbon atoms or an acylglycyl group, the acyl group of which has 1 to 6 carbon atoms, and its pharmacologically acceptable salts.

2. A process for isolating a purified polypeptide as claimed in claim 1, which comprises isolating the polypeptide from thymus with the aid of a combination of precipitation methods, membrane filtrations and chromatography, if appropriate converting the resulting peptide into its N-acyl or N-acylglycyl derivative in a manner which is known per se, and, if appropriate, converting the product into a salt.

3. The process as claimed in claim 2, wherein the glands are extracted with an aqueous solution of an inorganic salt having a buffering action in the pH range from 5.0 to 9.0, the insoluble material is separated off and the polypeptide is isolated from the resulting extract by separation processes which are known per se.

4. The process as claimed in claim 2, wherein the chromatography step comprises cation and/or anion exchange chromatography, chromatography on non-specific adsorbents, partition chromatography or a combination of two or more of these methods.

5. The process as claimed in claim 2, wherein an extract which has been subjected to heat-denaturing, the peptide as claimed in Claim 1 remaining in solution, whilst other proteins can be separated off by sedimentation or filtration, is used for the chromatography as claimed in claim 4.

6. The process as claimed in claim 4, wherein a material which, in the treatment of the extract as claimed in either of claims 3 or 5 by addition of water-miscible organic solvents, is separated off as a precipitate, is used for the chromatography.

7. The process as claimed in claim 4, wherein an extract which has been subjected to fractional precipitation by addition of water-soluble inorganic and/or organic salts, the peptide precipitating in certain concentration ranges, depending on the salt used, and being concentrated by sedimentation techniques, is used for the chromatography.

8. The process as claimed in claim 4, wherein an extract which, as a result of fractionation by means of membrane filtration and/or gel filtration, still contains only peptides which have a molecular weight below 10,000 Daltons, is used for the chromatography.

9. The process as claimed in claim 4, wherein an extract which has been obtained from a combination of two or more processes as claimed in any of claims 5 to 8 is used for the chromatography.

10. The use of the peptide as claimed in claim 1 as a therapeutic or prophylactic agent for regulating or stimulating the immunological system.

11. A medicament for regulating or stimulating the immunological system, which contains effective amounts of the polypeptide as claimed in claim 1 in the free form or in the form of a pharmacologically acceptable salt.

12. An aqueous sterile solution of a purified polypeptide as claimed in claim 1 in the free form or in the form of a pharmacologically acceptable salt, which contains an isotonic agent and, if appropriate, a preservative.

13. The peptide as claimed in claim 1 for use as a medicine.

## Claims for the Contracting State: AT

1. A process for isolating a purified polypeptide having an action on the immunological system, which has a molecular weight of 3,480, a UV absorption with a maximum at 205 to 210 nm, an isoelectric point at $3.95\pm0.15$ and the following aminoacid composition: 4 aspartic acid units, 3 threonine units, 3 serine units, 6 glutamic acid units, 1 glycine unit, 4 alanine units, 3 valine units, 1 isoleucine unit, 1 leucine unit, 5 lysine units and 1 arginine unit, the N-terminal, if appropriate, carrying an acyl group having 1 to 6 carbon atoms or an acylglycyl group, the acyl group of which has 1 to 6 carbon atoms, and its pharmacologically acceptable salts, which comprises isolating the polypeptide from thymus with the aid of a combination of precipitation methods, membrane filtrations and chromatography, if appropriate converting the resulting peptide into its N-acyl or N-acylglycl derivative in a manner which is known per se, and, if appropriate, converting the product into a salt.

2. The process as claimed in claim 1, wherein the glands are extracted with an aqueous solution of an inorganic salt having a buffering action in the pH range from 5.0 to 9.0, the insoluble material is separated off and the polypeptide is isolated from the resulting extract by separatin processes which are known per se.

3. The process as claimed in claim 1, wherein the chromatography step comprises cation and/or anion exchange chromatography, chromatography on non-specific adsorbents, partition chromatography or a combination of two or more of these methods.

4. The process as claimed in claim 1, wherein an extract which has been subjected to heat-denaturing, the peptide as claimed in claim 1 remaining in solution, whilst other proteins can be separated off by sedimentation or filtration, is used for the chromatography as claimed in claim 3.

5. The process as claimed in claim 3, wherein a material which, in the treatment of the extract as claimed in claims 2 and/or 4 by addition of water-miscible organic solvents, is separated off as a precipitate, is used for the chromatography.

6. The process as claimed in claim 3, wherein an extract which has been subjected to fractional precipitation by addition of water-soluble inorganic and/or organic salts, the peptide precipitating in certain concentration ranges, depending on the salt used, and being concentrated by sedimentation techniques, is used for the chromatography.

7. The process as claimed in claim 3, wherein an extract which, as a result of fractionation by means of membrane filtration and/or gel filtration, still contains only peptides which have a molecular weight below 10,000 Daltons, is used for the chromatography.

8. The process as claimed in claim 3, wherein an extract which has been obtained from a combination of two or more processes as claimed in claims 4 to 7 is used for the chromatography.

9. The process for isolating a purified polypeptide as claimed in any one of claims 1 to 8, wherein the peptide as defined in claim 1 is prepared for use as a medicine.

10. A process for preparing a medicament for regulating or stimulating the immunological system, which comprises bringing an effective amount of the polypeptide as claimed in claim 1 in the free form or in the form of a pharmacologically acceptable salt into an appropriate form.

11. A process for the preparation of an aqueous sterile solution of a purified polypeptide as claimed in claim 1 in the free form or in the form of a pharmacologically acceptable salt, which contains an isotonic agent and, if appropriate, a preservative, which comprises bringing these into solution.

FIG. 1

EP 0 101 063 B1

FIG. 2

FIG. 3

FIG. 4

FIG. 5